# EUROPEAN PATENT APPLICATION

(11) **EP 4 610 274 A1**
(43) Date of publication of application: **03.09.2025**
(21) Application number: 24736979.6
(22) Date of filing: 02.01.2024
(51) Int. Cl.: C07K 14/34, C12N 15/77, C12P 13/10, C12R 1/15

(54) **NOVEL BBD29_RS14450 VARIANT AND METHOD FOR PRODUCING L-ARGININE USING SAME**

(30) Priority: 30.12.2022 KR 20220191210
(71) Applicant: CJ CheilJedang Corporation, Seoul 04560 (KR)
(72) Inventor: LEE, Han Hyoung, Seoul 04560 (KR); KIM, Ju Eun, Seoul 04560 (KR); LEE, Zeewon, Seoul 04560 (KR); KIM, Ye-Eun, Seoul 04560 (KR)
(74) Representative: Meissner Bolte Partnerschaft mbB
(86) International application number: PCT/KR2024/000065
(87) International publication number: WO 2024/144382

(57) **Abstract**

Provided are a novel BBD29_RS14450 variant polypeptide; a polynucleotide encoding the variant polypeptide; a microorganism comprising any one or more of the variant polypeptide, the polynucleotide encoding the variant polypeptide, and a vector comprising the polynucleotide; and a method of producing L-arginine, the method comprising the step of culturing the microorganism in a medium.

## Description

### [Technical Field]

The present disclosure relates to a novel BBD29_RS14450 variant polypeptide; a polynucleotide encoding the variant polypeptide; a microorganism comprising any one or more of the variant polypeptide, the polynucleotide encoding the variant polypeptide, and a vector comprising the polynucleotide; and a method of producing L-arginine, the method comprising the step of culturing the microorganism in a medium.

### [Background Art]

L-arginine is used for medicinal purposes such as liver function enhancers, brain function enhancers, multi-amino acid preparations, etc., and is also a substance that has recently received attention in food applications such as fish cake additives, health beverage additives, salt substitutes for patients with hypertension, etc. Accordingly, in order to produce a high concentration of industrially applicable arginine, use of microorganisms has been continuously studied.

In order to produce L-amino acids and other useful substances, various studies have been conducted to develop high-efficiency production microorganisms. For example, for the production of L-arginine, target material-specific approaches are mainly used, such as a method of increasing the expression of a gene encoding an enzyme mainly involved in L-arginine biosynthesis in a strain of the genus *Corynebacterium,* or a method of deleting a gene unnecessary for the L-arginine biosynthesis (Korean Patent No. 10-1102263).

However, there is still a growing need for research on methods capable of efficiently producing L-arginine with a high yield.

### [Disclosure]

### [Technical Problem]

A problem to be solved in the present disclosure is to provide a novel BBD29_RS14450 variant polypeptide; a polynucleotide encoding the variant polypeptide; a microorganism comprising the variant polypeptide, the polynucleotide encoding the variant polypeptide, or a vector comprising the polynucleotide; and a method of producing L-arginine, the method comprising the step of culturing the microorganism in a medium.

### [Technical Solution]

One aspect of the present disclosure is to provide a BBD29_RS14450 variant polypeptide consisting of an amino acid sequence of SEQ ID NO: 1, in which leucine, an amino acid corresponding to position 49 of SEQ ID NO: 3, is substituted with proline.

Another aspect of the present disclosure is to provide a polynucleotide encoding the BBD29_RS14450 variant polypeptide of the present disclosure.

Still another aspect of the present disclosure is to provide a microorganism comprising any one or more of the BBD29_RS14450 variant polypeptide of the present disclosure, the polynucleotide encoding the same, and a vector comprising the polynucleotide.

Still another aspect of the present disclosure is to provide a method of producing L-arginine, the method comprising the step of culturing, in a medium, a microorganism comprising any one or more of the BBD29_RS14450 variant polypeptide of the present disclosure, the polynucleotide encoding the same, and a vector comprising the polynucleotide.

Still another aspect of the present disclosure is to provide a composition for producing L-arginine, the composition comprising the BBD29_RS14450 variant polypeptide of the present disclosure; a microorganism comprising any one or more of the variant polypeptide, the polynucleotide encoding the same, and a vector comprising the polynucleotide; or a combination thereof.

Still another aspect of the present disclosure is to provide use of the BBD29_RS14450 variant polypeptide of the present disclosure; and a microorganism comprising any one or more of the variant polypeptide, the polynucleotide encoding the same, and a vector comprising the polynucleotide in producing L-arginine.

### [Advantageous Effects]

When a microorganism comprising a variant polypeptide of the present disclosure is cultured, it is possible to produce L-arginine with a high yield, as compared to a microorganism having the existing unmodified polypeptide.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

One aspect of the present disclosure provides a BBD29_RS14450 variant polypeptide, in which an amino acid corresponding to position 49 of SEQ ID NO: 3 is substituted with a different amino acid.

In the present disclosure, the 'substitution with a different amino acid' is not limited as long as it is substitution with an amino acid different from the amino acid before substitution. Meanwhile, in the present disclosure, when it is expressed that 'a specific amino acid has been substituted', it is obvious that the amino acid is substituted with an amino acid different from the amino acid before substitution, even though it is not specifically stated that the amino acid has been substituted with a different amino acid.

In one embodiment of the aforementioned aspect, provided is a BBD29_RS14450 variant polypeptide consisting of an amino acid sequence of SEQ ID NO: 1, in which leucine, an amino acid corresponding to position 49 of SEQ ID NO: 3, is substituted with proline.

As used herein, the term "BBD29_RS14450 variant polypeptide" refers to a variant of a BBD29_RS14450 polypeptide comprising one or more amino acid substitutions in the amino acid sequence of the BBD29_RS14450 polypeptide; or a variant of a BBD29_RS14450 polypeptide comprising one or more amino acid substitutions in a parent sequence which is the amino acid sequence of the BBD29_RS14450 polypeptide.

A gene encoding the BBD29_RS14450 may be derived from a microorganism of the genus *Corynebacterium,* specifically, *Corynebacterium glutamicum,* but is not limited thereto.

The BBD29_RS14450 variant polypeptide of the present disclosure may have the amino acid sequence represented by SEQ ID NO: 1, or may essentially consist of the amino acid sequence.

Further, the BBD29_RS14450 variant polypeptide of the present disclosure may comprise an amino acid sequence having at least 70%, 75%, 80%, 85%, 90%, 95%, 96%, 97%, 98%, 99%, 99.5%, 99.7% or 99.9% or more homology or identity to the amino acid sequence represented by SEQ ID NO: 1, in which the amino acid corresponding to position 49 based on the amino acid sequence of SEQ ID NO: 3 is proline. It is also apparent that a variant polypeptide having an amino acid sequence having deletion, modification, substitution, conservative substitution, or addition of some sequence also falls within the scope of the present disclosure as long as the amino acid sequence has such homology or identity and exhibits efficacy corresponding to that of the variant polypeptide of the present disclosure.

For example, there are cases involving the addition or deletion of a sequence which does not alter the function of the variant of the present disclosure at the N-terminus of, C-terminus of, and/or inside the amino acid sequence, a naturally occurring mutation, a silent mutation thereof, or a conservative substitution.

The "conservative substitution" means substituting an amino acid with another amino acid having similar structural and/or chemical properties. Such amino acid substitution may generally occur based on similarity in the polarity, charge, solubility, hydrophobicity, hydrophilicity, and/or amphipathic nature of the residue. Usually, conservative substitution may hardly affect or not affect activity of proteins or polypeptides.

Further, the BBD29_RS14450 protein having the amino acid sequence of SEQ ID NO: 1 may be encoded by a polynucleotide having or comprising a nucleotide sequence of SEQ ID NO: 2 or a nucleotide sequence having 70% or more, 75% or more, 80% or more, 85% or more, 90% or more, 95% or more, 96% or more, 97% or more, 98% or more, or 99% or more, and less than 100% homology or identity to the sequence of SEQ ID NO: 2, or consisting of or essentially consisting of the nucleotide sequence of SEQ ID NO: 2 or a nucleotide sequence having 60% or more, 70% or more, 75% or more, 80% or more, 85% or more, 90% or more, 95% or more, 96% or more, 97% or more, 98% or more, or 98% or more, and less than 100% homology or identity to the sequence of SEQ ID NO: 2, but is not limited thereto.

As used herein, the term "variant" refers to a polypeptide in which one or more amino acids are conservatively substituted and/or modified so as to be different from the amino acid sequence of the variant before modification, while retaining functions or properties thereof. Such a variant may generally be identified by modifying one or more amino acids in the amino acid sequence of the polypeptide and evaluating the properties of the modified polypeptide. In other words, the ability of the variant may be increased, unchanged, or decreased, as compared to the polypeptide before modification. Further, some variants may comprise variants in which one or more portions, such as an N-terminal leader sequence or transmembrane domain, have been removed. Other variants may comprise variants in which a portion has been removed from the N- and/or C-terminus of a mature protein. The term "variant" may also be used interchangeably with a modification, modified polypeptide, modified protein, mutant, mutein, divergent, etc., and any term is not limited, as long as it is used in a sense of being mutated.

With respect to the objects of the present disclosure, the BBD29_RS14450 variant may be a polypeptide comprising the amino acid sequence represented by SEQ ID NO: 1, in which leucine, the amino acid corresponding to position 49 of the amino acid sequence of SEQ ID NO: 3, is substituted with proline.

The variant may also comprise deletion or addition of amino acids that have minimal influence on the properties and secondary structure of the polypeptide. For example, a signal (or leader) sequence which is co-translationally or post-translationally involved in translocation of the protein may be conjugated at the N-terminus of the variant. The variant may also be conjugated to other sequence or a linker for identification, purification, or synthesis.

As used herein, the term 'homology' or 'identity' refers to a degree of similarity between two given amino acid sequences or nucleotide sequences, and may be expressed as a percentage. The terms homology and identity may often be used interchangeably with each other.

The sequence homology or identity of a conserved polynucleotide or polypeptide is determined by standard alignment algorithms, and the default gap penalty established by a program to be used may be used together. Substantially, homologous or identical sequences may generally hybridize under moderately or highly stringent conditions to the entirety or a part of the sequence. It is apparent that hybridization also comprises hybridization with a polynucleotide comprising a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, the homology, similarity, or identity may be determined using BLAST or ClustalW of the National Center for Biotechnology Information.

The homology, similarity, or identity between polynucleotides or polypeptides may be determined by comparing sequence information using the GAP computer program as introduced by, for example, Needleman et al. (1970), J Mol Biol. 48:443, as disclosed by Smith and Waterman, Adv. Appl. Math (1981) 2:482. Briefly, the GAP program defines the same as the value obtained by dividing the number of similarly aligned symbols (i.e., nucleotides or amino acids) by the total number of the symbols in the shorter of the two sequences. The default parameters for the GAP program may comprise: (1) a binary comparison matrix (comprising a value 1 for identity and a value 0 for non-identity) and the weighted comparison matrix of Gribskov et al(1986) Nucl. Acids Res. 14: 6745 as disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

In one embodiment of the present disclosure, the variant of the present disclosure may have GntR family transcriptional regulator activity. Further, the variant of the present disclosure may have activity to increase the L-arginine-producing ability, as compared to the wild-type BBD29_RS14450 polypeptide. In the present disclosure, the sequence of the wild-type BBD29_RS14450 polypeptide may be obtained from a known database, NCBI's Genbank, etc.

As used herein, the "position N" may comprise position N and an amino acid position corresponding to the position N. Specifically, it may comprise an amino acid position corresponding to any amino acid residue in a mature polypeptide disclosed in a particular amino acid sequence. The particular amino acid sequence may be the amino acid sequence of SEQ ID NO: 3.

As used herein, the term "corresponding to" refers to an amino acid residue at a position listed in a polypeptide, or an amino acid residue that is similar, identical, or homologous to a residue listed in a polypeptide. Identifying the amino acid at the corresponding position may be determining a specific amino acid in a sequence that refers to a specific sequence. The "corresponding region" used herein generally refers to a similar or corresponding position in a related protein or reference protein.

For example, any amino acid sequence is aligned with SEQ ID NO: 3, and based on this, each amino acid residue of the amino acid sequence may be numbered by reference to the numerical position of the amino acid residue corresponding to the amino acid residue of SEQ ID NO: 3. For example, a sequence alignment algorithm such as that described herein may identify the position of an amino acid, or a position where a modification such as substitution, insertion or deletion occurs, compared to a query sequence (also referred to as a "reference sequence").

For such alignment, for example, Needleman-Wunsch algorithm (Needleman and Wunsch, 1970, J. Mol. Biol. 48: 443-453), Needleman program of EMBOSS package (EMBOSS: The European Molecular Biology Open Software Suite, Rice et al., 2000), Trends Genet. 16: 276-277) and the like may be used without being limited thereto, and sequence alignment programs, pairwise sequence comparison algorithm and the like known in the art may be appropriately used.

Another aspect of the present disclosure provides a BBD29_RS01115 variant polypeptide consisting of an amino acid sequence of SEQ ID NO: 5, in which threonine, an amino acid corresponding to position 11 of SEQ ID NO: 7, is substituted with isoleucine. The BBD29_RS01115 variant polypeptide of the present disclosure may have the amino acid sequence represented by SEQ ID NO: 5 or may essentially consist of the amino acid sequence.

Further, the BBD29_RS01115 variant polypeptide of the present disclosure may comprise an amino acid sequence having at least 70%, 75%, 80%, 85%, 90%, 95%, 96%, 97%, 98%, 99%, 99.5%, 99.7% or 99.9% or more homology or identity to the amino acid sequence represented by SEQ ID NO: 5, in which the amino acid corresponding to position 11 based on the amino acid sequence of SEQ ID NO: 7 is isoleucine.

Further, the BBD29_RS01115 protein having the amino acid sequence of SEQ ID NO: 5 may be encoded by a polynucleotide having, comprising, consisting of, or essentially consisting of a nucleotide sequence of SEQ ID NO: 6 or a nucleotide sequence having 70% or more, 75% or more, 80% or more, 85% or more, 90% or more, 95% or more, 96% or more, 97% or more, 98% or more, or 99% or more, and less than 100% homology or identity to the sequence of SEQ ID NO: 6, but is not limited thereto.

Still another aspect of the present disclosure provides a BBD29_RS02580 variant polypeptide consisting of an amino acid sequence of SEQ ID NO: 9, in which phenylalanine, an amino acid corresponding to position 280 of SEQ ID NO: 11, is substituted with valine. The BBD29_RS02580 variant polypeptide of the present disclosure may have the amino acid sequence represented by SEQ ID NO: 9 or may essentially consist of the amino acid sequence.

Further, the BBD29_RS02580 variant polypeptide of the present disclosure may comprise an amino acid sequence having at least 70%, 75%, 80%, 85%, 90%, 95%, 96%, 97%, 98%, 99%, 99.5%, 99.7% or 99.9% or more homology or identity to the amino acid sequence represented by SEQ ID NO: 9, in which the amino acid corresponding to position 280 based on the amino acid sequence of SEQ ID NO: 11 is valine.

Further, the BBD29_RS02580 protein having the amino acid sequence of SEQ ID NO: 9 may be encoded by a polynucleotide having, comprising, consisting of, or essentially consisting of a nucleotide sequence of SEQ ID NO: 10 or a nucleotide sequence having 70% or more, 75% or more, 80% or more, 85% or more, 90% or more, 95% or more, 96% or more, 97% or more, 98% or more, or 99% or more, and less than 100% homology or identity to the sequence of SEQ ID NO: 10, but is not limited thereto.

Still another aspect of the present disclosure provides a BBD29_RS07530 variant polypeptide consisting of an amino acid sequence of SEQ ID NO: 13, in which alanine, an amino acid corresponding to position 2 of SEQ ID NO: 17, is substituted with valine. The BBD29_RS07530 variant polypeptide of the present disclosure may have the amino acid sequence represented by SEQ ID NO: 13 or may essentially consist of the amino acid sequence.

Further, the BBD29_RS07530 variant polypeptide of the present disclosure may comprise an amino acid sequence having at least 70%, 75%, 80%, 85%, 90%, 95%, 96%, 97%, 98%, 99%, 99.5%, 99.7% or 99.9% or more homology or identity to the amino acid sequence represented by SEQ ID NO: 13, in which the amino acid corresponding to position 2 based on the amino acid sequence of SEQ ID NO: 17 is valine.

Further, the BBD29_RS07530 protein having the amino acid sequence of SEQ ID NO: 13 may be encoded by a polynucleotide having, comprising, consisting of, or essentially consisting of a nucleotide sequence of SEQ ID NO: 14 or a nucleotide sequence having 70% or more, 75% or more, 80% or more, 85% or more, 90% or more, 95% or more, 96% or more, 97% or more, 98% or more, or 99% or more, and less than 100% homology or identity to the sequence of SEQ ID NO: 14, but is not limited thereto.

Still another aspect of the present disclosure provides a BBD29_RS07530 variant polypeptide consisting of an amino acid sequence of SEQ ID NO: 15, in which alanine, the amino acid corresponding to position 2 of SEQ ID NO: 17, is substituted with valine, and amino acids corresponding to positions 79 to 82 are deleted. The BBD29_RS07530 variant polypeptide of the present disclosure may have the amino acid sequence represented by SEQ ID NO: 15 or may essentially consist of the amino acid sequence.

Further, the BBD29_RS07530 variant polypeptide of the present disclosure may comprise an amino acid sequence having at least 70%, 75%, 80%, 85%, 90%, 95%, 96%, 97%, 98%, 99%, 99.5%, 99.7% or 99.9% or more homology or identity to the amino acid sequence represented by SEQ ID NO: 15, in which the amino acid corresponding to position 2 based on the amino acid sequence of SEQ ID NO: 17 is valine, and the amino acids corresponding to positions 79 to 82 based on the amino acid sequence of SEQ ID NO: 17 are deleted.

Further, the BBD29_RS07530 protein having the amino acid sequence of SEQ ID NO: 15 may be encoded by a polynucleotide having, comprising, consisting of, or essentially consisting of a nucleotide sequence of SEQ ID NO: 16 or a nucleotide sequence having 70% or more, 75% or more, 80% or more, 85% or more, 90% or more, 95% or more, 96% or more, 97% or more, 98% or more, or 99% or more, and less than 100% homology or identity to the sequence of SEQ ID NO: 16, but is not limited thereto.

Still another aspect of the present disclosure provides a BBD29_RS07535 variant polypeptide consisting of an amino acid sequence of SEQ ID NO: 19, in which alanine, an amino acid corresponding to position 203 of SEQ ID NO: 21, is substituted with threonine. The BBD29_RS07535 variant polypeptide of the present disclosure may have the amino acid sequence represented by SEQ ID NO: 19 or may essentially consist of the amino acid sequence.

Further, the BBD29_RS07535 variant polypeptide of the present disclosure may comprise an amino acid sequence having at least 70%, 75%, 80%, 85%, 90%, 95%, 96%, 97%, 98%, 99%, 99.5%, 99.7% or 99.9% or more homology or identity to the amino acid sequence represented by SEQ ID NO: 19, in which the amino acid corresponding to position 203 based on the amino acid sequence of SEQ ID NO: 21 is threonine.

Further, the BBD29_RS07535 protein having the amino acid sequence of SEQ ID NO: 19 may be encoded by a polynucleotide having, comprising, consisting of, or essentially consisting of a nucleotide sequence of SEQ ID NO: 20 or a nucleotide sequence having 70% or more, 75% or more, 80% or more, 85% or more, 90% or more, 95% or more, 96% or more, 97% or more, 98% or more, or 99% or more, and less than 100% homology or identity to the sequence of SEQ ID NO: 20, but is not limited thereto.

Still another aspect of the present disclosure provides a BBD29_RS02010 variant polypeptide consisting of an amino acid sequence of SEQ ID NO: 97, in which arginine, an amino acid corresponding to position 349 of SEQ ID NO: 99, is substituted with glutamine. The BBD29_RS02010 variant polypeptide of the present disclosure may have the amino acid sequence represented by SEQ ID NO: 97 or may essentially consist of the amino acid sequence.

Further, the BBD29_RS02010 variant polypeptide of the present disclosure may comprise an amino acid sequence having at least 70%, 75%, 80%, 85%, 90%, 95%, 96%, 97%, 98%, 99%, 99.5%, 99.7% or 99.9% or more homology or identity to the amino acid sequence represented by SEQ ID NO: 97, in which the amino acid corresponding to position 349 based on the amino acid sequence of SEQ ID NO: 99 is glutamine.

Further, the BBD29_RS02010 protein having the amino acid sequence of SEQ ID NO: 97 may be encoded by a polynucleotide having, comprising, consisting of, or essentially consisting of a nucleotide sequence of SEQ ID NO: 98 or a nucleotide sequence having 70% or more, 75% or more, 80% or more, 85% or more, 90% or more, 95% or more, 96% or more, 97% or more, 98% or more, or 99% or more, and less than 100% homology or identity to the sequence of SEQ ID NO: 98, but is not limited thereto.

Still another aspect of the present disclosure provides a polynucleotide encoding the BBD29_RS14450 polypeptide variant of the present disclosure.

As used herein, the term "polynucleotide" is a DNA or RNA strand having a certain length or more as a polymer of nucleotides in which nucleotide monomers are connected in a long chain by covalent bonds, and more specifically, refers to a polynucleotide fragment encoding the variant polypeptide.

The polynucleotide encoding the BBD29_RS14450 variant polypeptide of the present disclosure may comprise a nucleotide sequence encoding the amino acid sequence represented by SEQ ID NO: 1. For example of the present disclosure, the polynucleotide of the present disclosure may have or comprise the nucleotide sequence of SEQ ID NO: 2. Further, the polynucleotide of the present disclosure may consist of or essentially consist of the nucleotide sequence of SEQ ID NO: 2.

The polynucleotide of the present disclosure may undergo various modifications in the coding region without changing the amino acid sequence of the variant of the present disclosure in consideration of codon degeneracy or the codons preferred in an organism in which the variant of the present disclosure is to be expressed. Specifically, the polynucleotide of the present disclosure may have or comprise a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 2, or may consist of or essentially consist of a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 2, but is not limited thereto. In this regard, in the sequence having such homology or identity, a codon encoding the amino acid corresponding to position 49 of SEQ ID NO: 1 may be one of codons encoding proline.

Further, the polynucleotide of the present disclosure may comprise a probe that may be prepared from a known gene sequence, for example, may comprise any sequence without limitation as long as it is a sequence that is able to hybridize with a complementary sequence to the entirety or a part of the polynucleotide sequence of the present disclosure under stringent conditions.

The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York,9.50-9.51, 11.7-11.8). For example, conditions in which polynucleotides having high homology or identity, i.e., polynucleotides having 70% or more, 75% or more, 80% or more, 85% or more, 90% or more, 95% or more, 96% or more, 97% or more, 98% or more, or 99% or more homology or identity are hybridized with each other while polynucleotides having homology or identity lower than the above are not hybridized with each other, or washing conditions of the common Southern hybridization, i.e., washing once, specifically, twice or three times at a salt concentration and a temperature corresponding to 60°C, 1XSSC, 0.1% SDS, specifically 60°C, 0.1XSSC, 0.1% SDS, and more specifically 68°C, 0.1XSSC, 0.1% SDS.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present disclosure may be detected using a hybridization condition comprising a hybridization step at a Tm value of 55°C and the above-described conditions. Further, the Tm value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (e.g., J. Sambrook et al., supra).

Still another aspect of the present disclosure provides a vector comprising the polynucleotide of the present disclosure. The vector may be an expression vector for expressing the polynucleotide in host cells (e.g., microorganisms), but is not limited thereto.

The vector of the present disclosure may comprise a DNA construct comprising a nucleotide sequence of a polynucleotide encoding a desired polypeptide which is operably linked to a suitable expression regulatory region (or expression control sequence) so that the desired polypeptide may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable host cell, and then replicate or function independently of the host genome, or may be integrated into the genome itself.

The vector used in the present disclosure is not particularly limited, but any vector known in the art may be used. Examples of commonly used vectors comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pBR system, pUC system, pBluescript II system, pGEM system, pTZ system, pCL system, pET system, etc. may be used as a plasmid vector. Specifically, pDZ, pDC, pDCM2 (Korean Patent Publication No. 10-2020-0136813, WO 2021-187781 A1), pACYC177, pACYC184, pCL, pECCG117, pUC19, pBR322, pMW118, pCC1BAC vector, etc. may be used.

For example, a polynucleotide encoding a desired polypeptide may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for the confirmation of chromosome insertion. The selection marker is for selecting the cells transformed with vectors, i.e., for confirming the insertion of a desired nucleic acid molecule, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide comprises DNA and/or RNA encoding a desired polypeptide. The polynucleotide may be introduced in any form as long as it may be introduced into a host cell and expressed. For example, the polynucleotide may be introduced into a host cell in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, as used herein, the term "operably linked" means that the polynucleotide sequence is functionally linked to a promoter sequence that initiates and mediates transcription of the polynucleotide encoding the desired variant of the present disclosure.

Still another aspect of the present disclosure provides a strain comprising the BBD29_RS14450 polypeptide variant of the present disclosure or the polynucleotide of the present disclosure.

The strain of the present disclosure may comprise any one or more of the BBD29_RS14450 variant polypeptide of the present disclosure, the polynucleotide encoding the polypeptide, and the vector comprising the polynucleotide of the present disclosure.

As used herein, the term "strain (or microorganism)" comprises all of wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism comprising genetic modification for production of a desired polypeptide, protein, or product. In the present disclosure, the "strain" and "microorganism" may be used interchangeably without limitation, with the same meaning.

The strain of the present disclosure may be a strain comprising any one or more of the BBD29_RS14450 variant polypeptide of the present disclosure, the polynucleotide of the present disclosure, and the vector comprising the polynucleotide of the present disclosure; a strain modified to express the BBD29_RS14450 variant polypeptide of the present disclosure or the polynucleotide of the present disclosure; a strain (e.g., recombinant strain) expressing the BBD29_RS14450 variant polypeptide of the present disclosure or the polynucleotide of the present disclosure; or a strain having the activity of the BBD29_RS14450 variant of the present disclosure (e.g., recombinant strain), but is not limited thereto.

The strain of the present disclosure may be a strain having an L-arginine-producing ability.

The strain of the present disclosure may be a microorganism naturally having the BBD29_RS14450 or the L-arginine-producing ability, or a microorganism prepared by introducing any one or more of the variant polypeptide of the present disclosure, the polynucleotide encoding the same, and the vector comprising the polynucleotide into a parent strain without the BBD29_RS14450 or the L-arginine-producing ability, and/or by providing the L-arginine-producing ability therefor, but is not limited thereto.

For example, the strain of the present disclosure may be a cell or microorganism that is transformed with the polynucleotide of the present disclosure or the vector comprising the polynucleotide encoding the variant polypeptide of the present disclosure to express the variant polypeptide of the present disclosure. With respect to the objects of the present disclosure, the strain of the present disclosure may comprise all microorganisms capable of producing L-arginine by comprising the variant polypeptide of the present disclosure. For example, the strain of the present disclosure may be a recombinant strain with the increased L-arginine-producing ability, in which the BBD29_RS14450 variant is expressed by introducing the polynucleotide encoding the variant polypeptide of the present disclosure into a natural wild-type microorganism or a microorganism producing L-arginine. The recombinant strain with the increased L-arginine-producing ability may be a microorganism in which the L-arginine-producing ability is increased, as compared to a natural wild-type microorganism or a BBD29_RS14450-unmodified microorganism (e.g., a microorganism expressing the wild-type BBD29_RS14450 (SEQ ID NO: 3) or a microorganism not expressing the variant (SEQ ID NO: 1) protein of the present disclosure), but is not limited thereto. For example, the BBD29_RS14450-unmodified microorganism which is a strain for comparing whether or not the L-arginine-producing ability is increased may be a strain KCCM10741P(KR 10-0791659 B1, EP 2041265 B1) or a CJ1R strain (KR 10-2022-0139085 A, WO 2022-216088 A1), but is not limited thereto.

For example, the recombinant strain having the increased production ability may have an increased L-arginine-producing ability of about 1% or more, about 2.5% or more, about 5% or more, about 7% or more, about 10% or more, about 12% or more, about 13% or more, about 14% or more, about 15% or more, about 17% or more, about 19% or more, or about 20% or more (the upper limit is not particularly limited, but may be, for example, about 200% or less, about 150% or less, about 100% or less, about 50% or less), as compared to the parent strain before modification or unmodified microorganism, but the increased amount is not limited thereto as long as the production ability has an increased amount of a + value, as compared to the production ability of the parent strain before modification or the unmodified microorganism. In another example, the recombinant strain having the increased production ability may have an increased L-arginine-producing ability of about 1.01 times or more, about 1.02 times or more, about 1.05 times or more, about 1.07 times or more, about 1.1 times or more, about 1.12 times or more, about 1.14 times or more, about 1.15 times or more, about 1.17 times or more, about 1.19 times or more, or about 1.20 times or more (the upper limit is not particularly limited, but may be, for example, about 10 times or less, about 5 times or less, about 3 times or less, about 2 times or less, or about 1.5 times or less), as compared to the parent strain before modification or the unmodified microorganism, but is not limited thereto. The term "about" refers to a range which comprises all of ±0.5, ±0.4, ±0.3, ±0.2, ±0.1, etc. and comprises all of the values that are equivalent or similar to those following the values, but the range is not limited thereto.

As used herein, the term "unmodified microorganism" does not exclude strains comprising mutations that may occur naturally in microorganisms, and may refer to a wild-type strain or natural strain itself or a strain before the trait is changed by genetic variation due to natural or artificial factors. For example, the unmodified microorganism may refer to a strain in which the BBD29_RS14450 variant polypeptide described herein is not introduced or a strain before introduction thereof. The "unmodified microorganism" may be used interchangeably with "strain before being modified", "microorganism before being modified", "unvaried strain", "unmodified strain", "unvaried microorganism", or "reference microorganism".

With regard to the microorganism according to any one of the aforementioned specific embodiments, the strain or microorganism of the present disclosure may be a microorganism of the *Corynebacterium* sp., *Escherichia* sp., *Erwinia* sp., *Serratia* sp., *Providencia* sp., *Pseudomonas* sp., *Leptospira* sp., *Salmonella* sp., *Brevibacteria* sp., *Hypomononas* sp., *Chromobacterium* sp., or *Norcardia* sp., or a microorganism of fungi or yeasts, specifically, a microorganism of *Corynebacterium* sp., but is not limited thereto.

For another example of the present disclosure, the strain or microorganism of the present disclosure may be a microorganism of *Corynebacterium* sp., specifically, *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

As used herein, the term "weakening" of activity of a polypeptide is a concept comprising both cases where the activity is decreased, as compared to the endogenous activity, or the activity is absent. The weakening may be used interchangeably with terms such as inactivation, deficiency, down-regulation, decrease, reduce, attenuation, etc.

The weakening may also comprise a case where the activity of the polypeptide itself is decreased or eliminated due to variation of the polynucleotide encoding the polypeptide, etc., as compared to the activity of the polypeptide originally possessed by the microorganism, a case where the overall polypeptide activity level and/or concentration (expression level) in the cell is low due to inhibition of the expression of the gene of the polynucleotide encoding the polypeptide or by inhibition of translation into the polypeptide, as compared to that of the natural strain, a case where the polynucleotide is not expressed at all, and/or a case where the polypeptide activity is absent even when the polynucleotide is expressed. The "endogenous activity" means the activity of a specific polypeptide originally possessed by the parent strain before change of the trait or a wild-type or unmodified microorganism when the trait is changed by genetic variation due to natural or artificial factors. This may be used interchangeably with "activity before modification". The fact that the activity of the polypeptide is "inactivated, deficient, decreased, down-regulated, reduced, or attenuated" as compared to the endogenous activity means that the activity of the polypeptide is lowered, as compared to the activity of a specific polypeptide originally possessed by the parent strain before change of the trait or the unmodified microorganism.

Such weakening of the activity of a polypeptide may be performed by any method known in the art, but the method is not limited thereto, and the weakening may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the polypeptide activity of the present disclosure may be:
1) deletion of the entirety or a part of the gene encoding the polypeptide;
2) modification of an expression regulatory region (or expression regulatory sequence) to decrease expression of the gene encoding the polypeptide;
3) modification of an amino acid sequence constituting the polypeptide to eliminate or weaken the activity of the polypeptide (e.g., deletion/substitution/addition of one or more amino acids in the amino acid sequence);
4) modification of a gene sequence encoding the polypeptide to eliminate or weaken the activity of the polypeptide (e.g., deletion/substitution/addition of one or more nucleic acid bases in a nucleic acid sequence of the polypeptide gene to encode the polypeptide that has been modified to eliminate or weaken the activity of the polypeptide);
5) modification of a nucleotide sequence encoding a start codon or 5'-UTR region of a gene transcript encoding the polypeptide;
6) introduction of an antisense oligonucleotide (e.g., antisense RNA) that complementarily binds to the transcript of the gene encoding the polypeptide;
7) addition of a sequence complementary to a Shine-Dalgarno sequence in front of the Shine-Dalgarno sequence of the gene encoding the polypeptide in order to form a secondary structure to which ribosome cannot be attached;
8) addition of a promoter to be transcribed in the opposite direction to the 3' end of the open reading frame (ORF) of the gene sequence encoding the polypeptide (reverse transcription engineering, RTE); or
9) a combination of two or more selected from 1) to 8), but is not particularly limited thereto.

For example,
1) the deletion of a part or the entirety of the gene encoding the polypeptide may be removal of the entire polynucleotide encoding the endogenous target polypeptide in the chromosome, replacement with a polynucleotide in which some nucleotides are deleted, or replacement with a marker gene.

Further, 2) the modification of the expression regulatory region (or expression regulatory sequence) may be occurrence of variation on the expression regulatory region (or expression regulatory sequence) due to deletion, insertion, non-conservative or conservative substitution, or a combination thereof, or replacement with a sequence exhibiting weaker activity. The expression regulatory region comprises a promoter, an operator sequence, a sequence encoding a ribosome binding site, and a sequence regulating the termination of transcription and translation, but is not limited thereto.

Further, 3) the modification of a nucleotide sequence encoding a start codon or 5'-UTR region of a gene transcript encoding the polypeptide may be, for example, replacing with a nucleotide sequence encoding another start codon having a lower polypeptide expression rate than the endogenous start codon, but is not limited thereto.

Further, 4) and 5) the modification of the amino acid sequence or polynucleotide sequence may be occurrence of variation on the amino acid sequence of the polypeptide or the polynucleotide sequence encoding the polypeptide due to deletion, insertion, or non-conservative or conservative substitution, or a combination thereof, or replacement with an amino acid sequence or a polynucleotide sequence which is improved to have weaker activity or an amino acid sequence or a polynucleotide sequence which is improved to be inactive such that the activity of the polypeptide is weakened, but is not limited thereto. For example, expression of the gene may be inhibited or weakened by introducing variation into the polynucleotide sequence and forming a stop codon, but is not limited thereto.

6) The introduction of an antisense oligonucleotide (e.g., antisense RNA) that complementarily binds to the transcript of the gene encoding the polypeptide, may refer to documents, for example, [Weintraub, H. et al., Antisense-RNA as a molecular tool for genetic analysis, Reviews - Trends in Genetics, Vol. 1(1) 1986].

7) The addition of a sequence complementary to a Shine-Dalgarno sequence in front of the Shine-Dalgarno sequence of the gene encoding the polypeptide in order to form a secondary structure to which ribosome cannot be attached may be to make mRNA translation impossible or to slow down the mRNA translation rate.

8) The addition of a promoter to be transcribed in the opposite direction to the 3' end of the open reading frame (ORF) of the gene sequence encoding the polypeptide (reverse transcription engineering, RTE) may be to weaken the activity by making an antisense nucleotide complementary to the transcript of the gene encoding the polypeptide.

As used herein, the term "enhancement" of activity of a polypeptide means that the activity of the polypeptide is increased as compared to the endogenous activity. The enhancement may be used interchangeably with terms such as activation, up-regulation, overexpression, increase, etc. Here, activation, enhancement, up-regulation, overexpression, and increase may comprise both exhibiting activity that was not originally possessed and exhibiting improved activity, as compared to the endogenous activity or activity before modification. The "endogenous activity" means the activity of a specific polypeptide originally possessed by a parent strain before change of the trait or an unmodified microorganism when the trait is changed by genetic variation due to natural or artificial factors. This may be used interchangeably with "activity before modification". The fact that the activity of the polypeptide is "enhanced", "up-regulated", "overexpressed", or "increased", as compared to the endogenous activity, means that the activity of the polypeptide is improved, as compared to the activity and/or concentration (expression level) of a specific polypeptide originally possessed by a parent strain before change of the trait or an unmodified microorganism.

The enhancement may be achieved through the introduction of a foreign polypeptide or the enhancement of endogenous activity and/or concentration (expression level) of the polypeptide. The activity enhancement of the polypeptide may be confirmed by an increase in the degree of activity and the expression level of the corresponding polypeptide or in the amount of a product produced from the corresponding polypeptide.

For the activity enhancement of the polypeptide, various methods well known in the art may be applied, and the method is not limited as long as the activity of the target polypeptide may be enhanced, as compared to that of the microorganism before modification. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be:
1) increase in the intracellular copy number of the polynucleotide encoding the polypeptide;
2) replacement of a gene expression regulatory region on a chromosome encoding the polypeptide with a sequence exhibiting strong activity;
3) modification of a nucleotide sequence encoding a start codon or a 5'-UTR region of a gene transcript encoding the polypeptide;
4) modification of the amino acid sequence of the polypeptide to enhance the activity of the polypeptide;
5) modification of the polynucleotide sequence encoding the polypeptide to enhance the activity of the polypeptide (e.g., modification of the polynucleotide sequence of the polypeptide gene to encode the polypeptide that has been modified to enhance the activity of the polypeptide);
6) introduction of a foreign polypeptide exhibiting the activity of the polypeptide or a foreign polynucleotide encoding the polypeptide;
7) codon optimization of a polynucleotide encoding the polypeptide;
8) analysis of the tertiary structure of the polypeptide to select the exposed site and to perform modification or chemical modification of the exposed site; or
9) a combination of two or more selected from 1) to 8), but is not particularly limited thereto.

More specifically,
1) the increase in the intracellular copy number of the polynucleotide encoding the polypeptide may be achieved by the introduction of, into a host cell, a vector which may replicate and function independently of the host and to which the polynucleotide encoding the corresponding polypeptide is operably linked. Alternatively, the increase may be achieved by the introduction of one copy or two or more copies of the polynucleotide encoding the corresponding polypeptide into a chromosome of a host cell. The introduction into the chromosome may be performed by introducing, into a host cell, a vector capable of inserting the polynucleotide into a chromosome of the host cell, but is not limited thereto. The vector is as described above.
2) The replacement of a gene expression regulatory region (or expression control sequence) on a chromosome encoding the polypeptide with a sequence exhibiting strong activity may be, for example, occurrence of variation on a sequence due to deletion, insertion, non-conservative or conservative substitution, or a combination thereof, or replacement with a sequence exhibiting stronger activity such that the activity of the expression regulatory region is further enhanced. The expression regulatory region may comprise, but is not particularly limited to, a promoter, an operator sequence, a sequence encoding a ribosome binding site, a sequence controlling the termination of transcription and translation, etc. For example, the replacement may be to replace the original promoter with a strong promoter, but is not limited thereto.
   Examples of known strong promoters comprise CJ1 to CJ7 promoters (US Patent No. 7662943 B2), a lac promoter, a trp promoter, a trc promoter, a tac promoter, a lambda phage PR promoter, a PL promoter, a tet promoter, a gapA promoter, an SPL7 promoter, an SPL13(sm3) promoter (US Patent No. 10584338 B2), an O2 promoter (US Patent No. 10273491 B2), a tkt promoter, an yccA promoter, etc., but is not limited thereto.
3) The modification of a nucleotide sequence encoding a start codon or a 5'-UTR region of a gene transcript encoding the polypeptide may be, for example, substitution with a nucleotide sequence encoding another start codon having a higher polypeptide expression rate as compared to an endogenous start codon, but is not limited thereto.
4) and 5) The modification of the amino acid sequence or the polynucleotide sequence may be occurrence of variation on the sequence due to deletion, insertion, non-conservative or conservative substitution of the amino acid sequence of the polypeptide or the polynucleotide sequence encoding the polypeptide, or a combination thereof, or replacement with an amino acid sequence or polynucleotide sequence which is improved to exhibit stronger activity or an amino acid sequence or polynucleotide sequence which is improved to be more active such that the activity of the polypeptide is enhanced, but is not limited thereto. The replacement may be specifically performed by inserting a polynucleotide into a chromosome by homologous recombination, but is not limited thereto. The vector used here may further comprise a selection marker for the confirmation of chromosome insertion. The selection marker is the same as described above.
6) The introduction of a foreign polynucleotide exhibiting the activity of the polypeptide may be introduction of, into a host cell, a foreign polynucleotide encoding a polypeptide exhibiting activity identical or similar to that of the polypeptide. There is no limitation on its origin or sequence as long as the foreign polynucleotide exhibits activity identical or similar to that of the polypeptide. The method used in the introduction may be performed by appropriately selecting a known transformation method by those skilled in the art. As the introduced polynucleotide is expressed in the host cell, the polypeptide may be produced, and the activity thereof may be increased.
7) The codon optimization of the polynucleotide encoding the polypeptide may be codon optimization of an endogenous polynucleotide so as to increase transcription or translation in a host cell, or codon optimization of a foreign polynucleotide so as to perform optimized transcription and translation in a host cell.
8) The analysis of the tertiary structure of the polypeptide to select and to modify or chemically modify the exposed site may be, for example, to determine a template protein candidate according to the degree of similarity of the sequence by comparing the sequence information of a polypeptide to be analyzed with a database storing the sequence information of known proteins, to confirm the structure based on this, and to modify or chemically modify the exposed site to be modified or chemically modified.

Such enhancement of the polypeptide activity may be an increase in the activity or concentration expression level of the corresponding polypeptide, based on the activity or concentration of the polypeptide expressed in a wild-type or a microbial strain before modification, or an increase in the amount of a product produced from the corresponding polypeptide, but is not limited thereto.

In the microorganism of the present disclosure, partial or entire modification of the polynucleotide may be induced by (a) homologous recombination using a vector for chromosome insertion in the microorganism or genome editing using engineered nuclease (e.g., CRISPR-Cas9) and/or (b) treatment with light such as ultraviolet rays and radiation, and/or chemicals, but is not limited thereto. A method of modifying a part or the entirety of the gene may comprise a method of using a DNA recombination technology. For example, by introducing a nucleotide sequence or vector comprising a nucleotide sequence homologous to the target gene into the microorganism to cause homologous recombination, a part or the entirety of the gene may be deleted. The nucleotide sequence or vector to be introduced may comprise a dominant selection marker, but is not limited thereto.

The strain and microorganism of the present disclosure may comprise any one or more of the BBD29_RS14450 variant polypeptide of the present disclosure, the polynucleotide encoding the same, and the vector comprising the polynucleotide, while further comprising any one or more of any one or more variant polypeptides of the BBD29_RS01115 variant polypeptide, the BBD29_RS02580 variant polypeptide, two kinds of BBD29_RS07530 variant polypeptides (e.g., SEQ ID NO: 13, SEQ ID NO: 15), the BBD29_RS07535 variant polypeptide, and the BBD29_RS02010 variant polypeptide, the polynucleotide encoding the same, and the vector comprising the polynucleotide.

In the microorganism of the present disclosure, the variant polypeptide, polynucleotide, L-arginine, etc. are as described in other aspects.

Still another aspect of the present disclosure provides a method of producing an L-amino acid, the method comprising the step of culturing, in a medium, a *Corynebacterium glutamicum* strain comprising the BBD29_RS14450 variant polypeptide of the present disclosure or the polynucleotide of the present disclosure.

The method of producing an L-amino acid of the present disclosure may comprise the step of culturing, in a medium, a strain comprising the variant of the present disclosure, the polynucleotide of the present disclosure, or the vector of the present disclosure.

Further, the L-amino acid of the present disclosure may be L-arginine.

As used herein, the term "culturing" refers to growing the strain of the present disclosure in appropriately adjusted environment conditions. The culture procedure of the present disclosure may be performed according to appropriate media or culture conditions known in the art. Such culturing process may be easily adjusted for use by a person skilled in the art according to the selected strain. Specifically, the culturing may be in a batch type, a continuous type, and/or a fed-batch type, but is not limited thereto.

As used herein, the "medium" refers to a mixture comprising, as main ingredients, nutrient materials required for culturing the strain of the present disclosure, wherein the medium supplies nutrient materials comprising water essential for survival and growth, growth factors, etc. Specifically, as for the media and other culture conditions used for culturing the strain of the present disclosure, any medium that is commonly used for culturing microorganisms may be used without particular limitation. However, the strain of the present disclosure may be cultured under aerobic conditions in a common medium comprising appropriate carbon sources, nitrogen sources, phosphorus sources, inorganic compounds, amino acids, and/or vitamins, while controlling the temperature, pH, etc.

Specifically, a culture medium for strain (particularly, *Corynebacterium*) of the present disclosure may be found in a literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphorus sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These constituent ingredients or precursors may be added to the medium in a batch or continuous manner. However, the present disclosure is not limited thereto.

Further, pH of the medium may be adjusted by adding compounds, such as ammonium hydroxide, potassium hydroxide, ammonia, phosphoric acid, and sulfuric acid, to the medium in an appropriate manner during culturing of the strain of the present disclosure. In addition, an anti-foaming agent, such as a fatty acid polyglycol ester, may be used to suppress foam formation during culturing. In addition, oxygen or oxygen-comprising gas may be injected into the medium to maintain the aerobic state of the medium, or no gas may be injected or nitrogen, hydrogen or carbon dioxide gas may be injected to maintain the anaerobic or non-aerobic state of the medium, but is not limited thereto.

In the culturing of the present disclosure, the culture temperature may be maintained at 20°C to 45°C, specifically 25°C to 40°C, and the culturing may be performed for about 10 hours to 160 hours, but is not limited thereto.

The L-amino acid produced by culturing of the present disclosure may be released into the medium or may remain in cells.

The method of producing an L-amino acid of the present disclosure may further comprise the steps of preparing the strain of the present disclosure, preparing a medium for culturing the strain, or a combination of these steps (regardless of the order, in any order), for example, before the culturing step.

The method of producing an L-amino acid of the present disclosure may further comprise the step of recovering the L-amino acid from the medium resulting from the culturing (medium in which culturing has been performed) or the strain of the present disclosure. The recovering step may be further comprised after the culturing step.

The recovering may be collecting a desired L-amino acid by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, HPLC**,** and a combination of these methods may be used, and the desired L-amino acid may be recovered from the medium or microorganism by using an appropriate method known in the art.

In addition, the method of producing an L-amino acid of the present disclosure may further comprise a purification step. The purification may be performed by using an appropriate method known in the art. In an exemplary embodiment, when the method of producing an L-amino acid of the present disclosure comprises both the recovering step and the purification step, the recovering step and the purification step may be performed continuously or discontinuously regardless of the order, or may be performed simultaneously or integrated into one step, but is not limited thereto.

In the method of the present disclosure, the variant, the polynucleotide, the vector, and the strain, etc. are as described in other aspects.

Still another aspect of the present disclosure provides a composition for producing an L-amino acid, the composition comprising the BBD29_RS14450 variant polypeptide of the present disclosure; the polynucleotide encoding the variant polypeptide; the vector comprising the polynucleotide; the strain comprising any one or more of the BBD29_RS14450 variant polypeptide of the present disclosure, the polynucleotide of the present disclosure, and the vector comprising the polynucleotide; a medium in which the strain is cultured; or a combination of two or more thereof.

The composition of the present disclosure may further comprise any appropriate excipient that is commonly used in the composition for producing amino acids, and such excipients may comprise, for example, a preserving agent, a wetting agent, a dispersing agent, a suspending agent, a buffer, a stabilizing agent, an isotonic agent, etc., but are not limited thereto.

In the composition of the present disclosure, the variant, the polynucleotide, the vector, the strain, the medium, and the L-amino acid, etc. are as described in other aspects.

Still another aspect of the present disclosure provides use of the BBD29_RS14450 variant polypeptide of the present disclosure; and the microorganism comprising any one or more of the BBD29_RS14450 variant polypeptide, the polynucleotide encoding the same, and the vector comprising the polynucleotide in producing L-arginine.

The variant polypeptide of the present disclosure, the polynucleotide, the vector, the microorganism, and the L-arginine, etc. are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Preparation of Mutant Strains with Increased Arginine-Producing Ability through Artificial Mutation

In order to select mutant strains with increased arginine-producing ability, random mutations were induced on the genome using UV irradiation (Ultraviolet mutation). An arginine-producing strain KCCM10741P (Korean Patent No. 10-0791659, EP 2041265 B1) was diluted and spread on a complex plate medium comprising agar, irradiated with UV, and cultured for 48 hours at 30°C to obtain mutant colonies. About 20,000 colonies thus obtained were screened and evaluated under 96-deep well plate conditions. A composition of the complex plate medium mentioned above is as follows.

### <Complex plate medium (pH 7.0)>

10 g of glucose, 10 g of peptone, 5 g of beef extract, 5 g of yeast extract, 18.5 g of brain heart infusion, 2.5 g of NaCl, 2 g of urea, 91 g of sorbitol, 20 g of agar (based on 1 liter of distilled water)

### Example 2. Screening and Evaluation of Mutant Strains with Increased Arginine-Producing Ability

About 20,000 colonies obtained in Example 1 were inoculated into 300 µl of a selection medium below and cultured in a 96-deep well plate at 30°C and 1,000 rpm for about 24 hours.

### <Selection medium (pH 8.0)>

10 g of glucose, 5.5 g of ammonium sulfate, 1.2 g of MgSO₄7H₂O, 0.8 g of KH₂PO₄, 16.4 g of K₂HPO₄, 100 µg of biotin, 1 mg of thiamine HCl, 2 mg of calcium-pantothenic acid, 2 mg of nicotinamide (based on 1 liter of distilled water)

A ninhydrin method was used to analyze the production amount of L-arginine produced in the culture medium (Moore, S., Stein, W. H., Photometric ninhydrin method for use in the chromatography of amino acids. J. Biol. Chem.1948, 176, 367-388).

To this end, after the culture of each mutant strain was completed, 10 µl of the culture supernatant and 190 µl of a ninhydrin reaction solution were reacted at 65°C for 30 minutes, and absorbance of the reaction solution was measured at a wavelength of 570 nm using a spectrophotometer. About 30 colonies of mutant strains showing high absorbance, as compared to the control *Corynebacterium glutamicum* KCCM10741P strain, were selected. It was confirmed that the other colonies showed similar or lowered absorbance, as compared to the *Corynebacterium glutamicum* KCCM10741P strain used as the control.

30 types of strains selected above were re-cultured using the same method and the ninhydrin reaction was repeated to select the top 10 mutant strains with the most improved L-arginine-producing ability, as compared to the parent strain, *Corynebacterium glutamicum* KCCM10741P strain.

### Example 3: Analysis of L-Arginine-Producing Ability of Selected Random Mutant Strains

In order to finally select strains, in which the L-arginine-producing ability was reproducibly increased, from 10 types of the mutant strains selected in Example 2, flask culture was performed using the following medium. After the culture was completed, the concentration of L-arginine in the culture medium was analyzed using HPLC, and the concentration of L-arginine produced in each mutant strain is shown in Table 1 below.

### <Production medium (pH 7.0)>

6% glucose, 3% ammonium sulfate, 0.1% potassium phosphate monobasic, 0.2% magnesium sulfate heptahydrate, 1.5% corn steep liquor (CSL), 1% NaCl, 0.5% yeast extract, 100 mg/l biotin, 3% CaCO₃ (based on 1 liter of distilled water).

**[Table 1]**

| No. | Strain | L-arginine (g/l) | | |
|---|---|---|---|---|
| | | Medium 1 | Medium 2 | Mean |
| Control group | KCCM10741P | 2.9 | 3.1 | 3.0 |
| 1 | KCCM10741P/ UV-1 | 3.2 | 3.3 | 3.3 |
| 2 | KCCM10741P/ UV-2 | 3.0 | 3.1 | 3.1 |
| 3 | KCCM10741P/ UV-3 | 3.1 | 3.1 | 3.1 |
| 4 | KCCM10741P/ UV-4 | 3.3 | 3.4 | 3.4 |
| 5 | KCCM10741P/ UV-5 | 3.4 | 3.4 | 3.4 |
| 6 | KCCM10741P/ UV-6 | 3.2 | 3.4 | 3.3 |
| 7 | KCCM10741P/ UV-7 | 3.6 | 3.6 | 3.6 |
| 8 | KCCM10741P/ UV-8 | 3.4 | 3.2 | 3.3 |
| 9 | KCCM10741P/ UV-9 | 3.3 | 3.5 | 3.4 |
| 10 | KCCM10741P/ UV-10 | 3.4 | 3.2 | 3.3 |

From 10 types of the selected mutant strains, KCCM10741P/UV-7 was finally selected, which showed the greatest improvement in the L-arginine-producing ability. The results confirmed that the KCCM10741P/UV-7 strain showed 20% increase in the arginine-producing ability, as compared to the control KCCM10741P strain, indicating improvement in the arginine yield.

### Example 4: Identification of Cause of Increased L-Arginine-Producing Ability in Final Selected Strain

The mutant strain KCCM10741P/UV-7 which was finally selected from Example 3 was subjected to NGS sequence analysis, and compared with the parent strain KCCM10741P strain and the wild-type *Corynebacterium glutamicum* ATCC13869 strain. Genes with changes in the protein sequence due to nucleotide sequence mutations were identified, and each gene substitution types are shown in Table 2 below.

Among the mutation types, the most common mutation type was confirmed to be introduction of the amino acid mutation due to nucleotide sequence substitution, which was 6. BBD29_RS07530 was confirmed to be a variant protein in which 4 amino acids were deleted by additionally deleting 12 bp of a nucleotide sequence from positions 233 to 245, in addition to the nucleotide sequence substitution as shown in Table 2 below.

**[Table 2]**

| Target gene | | Mutation site | |
|---|---|---|---|
| | | NT | AA |
| 1 | BBD29_RS01115 | C32T | T11I |
| 2 | BBD29_RS02580 | T838G | F280V |
| 3 | BBD29_RS07530 | C5T | A2V |
| 4 | | △12bp (233-245) | △PVEA (79-82) |
| 5 | BBD29_RS07535 | G607A | A203T |
| 6 | BBD29_RS14450 | T146C | L49P |
| 7 | BBD29_RS02010 | G1046A | R349Q |

### Example 5: Construction of Recombinant Vector to Examine Arginine-Producing Ability of Identified Genetic Mutation

In order to examine the effect of the protein variants of the KCCM10741P/UV-7 strain identified in Example 4 on the arginine-producing ability of the microorganism, a recombinant vector capable of inducing mutations (substitution mutation or deletion) in each protein was constructed. The recombinant vector was constructed, based on a pDCM2 (Korean Patent Publication No. 10-2020-0136813, WO 2021-187781 A1) plasmid, and used to replace a gene in the chromosome of a *Corynebacterium* strain.

gDNA (genomic DNA) of KCCM10741P/UV-7 selected in Example 3 was used as a template, and the upstream and downstream gene fragments based on the mutation site were obtained through PCR in order to introduce each mutant sequence. The name of the constructed vector and the sequence information of the used primers are listed in Table 3 below. With regard to each gene, PCR was performed using the F1 and R2 primer pair to obtain the upstream gene fragment based on the mutation site, and the F3 and R4 primer pair to obtain the downstream fragment. The PCR was performed by denaturation at 94°C for 5 minutes, followed by 30 cycles of 94°C for 30 seconds; 55°C for 30 seconds; and 72°C for 1 minute and 30 seconds, and then at 72°C for 5 minutes. The two fragments thus obtained, after DNA purification, were ligated (fusion cloning) to a pDCM2 plasmid treated with Smal restriction enzyme using an In-Fusion^{®} HD cloning kit (Clontech). The resulting vectors were named pDCM2-BBD29_RS01115 (C32T), pDCM2-BBD29_RS02580 (T838G), pDCM2-BBD29_RS07530 (C5T), pDCM2-BBD29_RS07530 (△233-245), pDCM2-BBD29_RS07535 (G607A), pDCM2-BBD29_RS14450 (T146C), and pDCM2-BBD29_RS02010 (G1046A).

**[Table 3]**

| SEQ ID NO. | Name of constructed vector | Primer No. | Sequence (5'-3') | PCR size |
|---|---|---|---|---|
| 45 | pDCM2-BBD29_RS01115 (C32T) | F1 | TGAATTCGAGCTCGGTACCCTGAGGTACGCAAA CTTTC | 531 |
| 46 | | R2 | CTGCTGCGGAATGTACCTGTCAGTACTT | |
| 47 | | F3 | GACAGGTACATTCCGCAGCAGCGCACGC | 530 |
| 48 | | R4 | GTCGACTCTAGAGGATCCCCGAAATCGTGGATG ATTTG | |
| 49 | pDCM2-BBD29_RS02580 (T838G) | F1 | TGAATTCGAGCTCGGTACCCCATCGCTGCGTAA GGAAT | 531 |
| 50 | | R2 | GCCACTGGGACAGGTGAGGTATCAGCGA | |
| 51 | | F3 | TACCTCACCTGTCCCAGTGGCTGCCTAC | 531 |
| 52 | | R4 | GTCGACTCTAGAGGATCCCCTGAAGAATCCCAT GACCA | |
| 53 | pDCM2-BBD29_RS07530 (C5T) | F1 | TGAATTCGAGCTCGGTACCCCCACTGGCGTTC AAGATG | 531 |
| 54 | | R2 | ACGGTAAATAACCATATGTTTTCCTTCC | |
| 55 | | F3 | AAACATATGGTTATTTACCGTAAGCTTG | 531 |
| 56 | | R4 | GTCGACTCTAGAGGATCCCCTGCTGCCAAAATT CACGA | |
| 57 | pDCM2-BBD29_RS07530 (△233-245) | F1 | TGAATTCGAGCTCGGTACCCCCACTGGCGTTC AAGATG | 760 |
| 58 | | R2 | GCTCAACAGGTGCCTCGACTGGAGCCTC | |
| 59 | | F3 | CCAGTCGAGGCACCTGTTGAGCAGGCAC | 303 |
| 60 | | R4 | GTCGACTCTAGAGGATCCCCTGCTGCCAAAATT CACGA | |
| 61 | pDCM2-BBD29_RS07535 (G607A) | F1 | TGAATTCGAGCTCGGTACCCGAGCACTCACCG CGGCAT | 531 |
| 62 | | R2 | GTGTTGTACGTCTTGAGTGAACCCATGG | |
| 63 | | F3 | TTCACTCAAGACGTACAACACCTCCGGA | 531 |
| 64 | | R4 | GTCGACTCTAGAGGATCCCCTTAGATATGTTTGT TGTG | |
| 77 | pDCM2-BBD29_RS14450 (T146C) | F1 | TGAATTCGAGCTCGGTACCCCGATTTCTAATTG GGCAC | 481 |
| 78 | | R2 | AAGGAGGGTCGGGCCGTTGAGTGCGGTG | |
| 79 | | F3 | CTCAACGGCCCGACCCTCCTTGTCGAAG | 496 |
| 80 | | R4 | GTCGACTCTAGAGGATCCCCATCACCTTGCTCG GTGTC | |
| 93 | pDCM2-BBD29_RS02010 (G1046A) | F1 | TGAATTCGAGCTCGGTACCCCAAGGTTCTGGTC ACGGG | 531 |
| 94 | | R2 | GCTGACAGCCTGAGAGAATACCGGAGAT | |
| 95 | | F3 | GTATTCTCTCAGGCTGTCAGCTGTGGAA | 531 |
| 96 | | R4 | GTCGACTCTAGAGGATCCCCTGACGGGGTTGA GCCAAG | |

### Example 6: Preparation of Microorganism Expressing Protein Variant and Evaluation of L-Arginine-Producing Ability

7 types of the vectors constructed in Example 5 were each transformed into *Corynebacterium glutamicum* CJ1R (Korean Patent Application Publication No. 10-2022-0139085, WO 2022-216088 A1). The corresponding vector and the host strain were transformed by electroporation (Appl. Microbiol. Biotechnol. (1999) 52:541-545), and a strain in which genetic mutation was inserted was obtained through a secondary crossover. The mutant strains thus obtained were named CJ1R-BBD29_RS01115 (C32T), CJ1R-BBD29_RS02580 (T838G), CJ1R-BBD29_RS07530 (C5T), CJ1R-BBD29_RS07530 (△233-245), CJ1R-BBD29_RS07535 (G607A), CJ1R-BBD29_RS14450 (T146C), and CJ1R-BBD29_RS02010 (G1046A), respectively.

In order to compare the L-arginine-producing abilities of the genetic mutation-introduced strains, the strains were cultured using the following method, and the concentration of L-arginine in the culture medium was analyzed.

The parent strain, *Corynebacterium glutamicum* CJ1R strain, and a total of 8 types of the transformed strains were each inoculated into a 250-ml corner-baffled flask comprising 25 ml of the following seed medium, and cultured at 30°C for 20 hours with shaking at 200 rpm. Thereafter, 1 ml of the seed culture medium was inoculated into a 250-ml corner-baffled flask comprising 24 ml of a production medium, and cultured at 30°C for 72 hours with shaking at 200 rpm. The compositions of the seed medium and the production medium are as follows, respectively.

### <Seed medium (pH 7.2)>

20 g of glucose, 45 g of ammonium sulfate, 2 g of magnesium sulfate heptahydrate, 2 g of potassium phosphate monobasic, 10 g of ammonium chloride, 0.01 mg of biotin, 0.1 mg of thiamine-HCl, 2 mg of calcium pantothenate, 3 mg of nicotinamide, 10 mg of iron sulfate, 10 mg of manganese sulfate, 0.02 mg of zinc sulfate, 0.5 mg of copper sulfate (based on 1 liter of distilled water)

### <Production medium (pH 7.2)>

60 g of glucose, 45 g of ammonium sulfate, 2 g of magnesium sulfate heptahydrate, 2 g of potassium phosphate monobasic, 10 g of ammonium chloride, 0.01 mg of biotin, 0.1 mg of thiamine-HCl, 2 mg of calcium pantothenate, 3 mg of nicotinamide, 10 mg of iron sulfate, 10 mg of manganese sulfate, 0.02 mg of zinc sulfate, 0.5 mg of copper sulfate, 30 g of calcium carbonate (based on 1L of distilled water)

After the culture was completed, the L-arginine-producing ability (concentration) was analyzed using HPLC (Waters 2478), and the analyzed concentrations of L-arginine are shown in Table 4 below.

**[Table 4]**

| Strain | | L-arginine (g/l) | |
|---|---|---|---|
| | | 3BT mean | Production ability |
| Control group | CJ1R | 2.1 | 100% |
| 1 | CJ1R-BBD29 RS01115 (C32T) | 2.2 | 105% |
| 2 | CJ1R-BBD29 RS02580 (T838G) | 2.1 | 100% |
| 3 | CJ1R-BBD29_RS07530 (C5T) | 2.2 | 105% |
| 4 | CJ1R-BBD29_RS07530 (△233-245) | 2.1 | 100% |
| 5 | CJ1R-BBD29_RS07535 (G607A) | 2.2 | 105% |
| 6 | CJ1R-BBD29_RS14450 (T146C) | 2.4 | 114% |
| 7 | CJ1R-BBD29_RS02010 (G1046A) | 2.4 | 114% |

As a result, it was confirmed that the L-arginine-producing ability of the variant-introduced strain CJ1R-BBD29_RS14450 (T146C) increased by 14%, as compared to the parent strain CJ1R, showing 114% L-arginine-producing ability.

The above results confirmed that the BBD29_RS14450 (T146C) mutation of the present disclosure increased the L-arginine-producing ability of the microorganism. Further, the result of the 20% increased production ability of the KCCM10741P/UV-7 mutant strain in Example 3 is attributed to the combined effect of the above mutations.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A BBD29_RS14450 variant polypeptide consisting of an amino acid sequence of SEQ ID NO: 1, wherein leucine, an amino acid corresponding to position 49 of SEQ ID NO: 3, is substituted with proline.

2. A polynucleotide encoding the variant polypeptide of claim 1.

3. The polynucleotide of claim 2, wherein the polynucleotide consists of a nucleotide sequence of SEQ ID NO: 2.

4. A *Corynebacterium glutamicum* strain comprising any one or more of the variant polypeptide of claim 1 and a polynucleotide encoding the variant polypeptide.

5. The *Corynebacterium glutamicum* strain of claim 4, wherein the strain further comprises any one or more of any one or more variant polypeptides of the following (a) to (f) and polynucleotides encoding the variant polypeptides:
(a) a BBD29_RS01115 variant polypeptide consisting of an amino acid sequence of SEQ ID NO: 5, wherein threonine, an amino acid corresponding to position 11 of SEQ ID NO: 7, is substituted with isoleucine;
(b) a BBD29_RS02580 variant polypeptide consisting of an amino acid sequence of SEQ ID NO: 9, wherein phenylalanine, an amino acid corresponding to position 280 of SEQ ID NO: 11, is substituted with valine;
(c) a BBD29_RS07530 variant polypeptide consisting of an amino acid sequence of SEQ ID NO: 13, wherein alanine, an amino acid corresponding to position 2 of SEQ ID NO: 17, is substituted with valine;
(d) a BBD29_RS07530 variant polypeptide consisting of an amino acid sequence of SEQ ID NO: 15, wherein alanine, an amino acid corresponding to position 2 of SEQ ID NO: 17, is substituted with valine, and amino acids corresponding to positions 79 to 82 are deleted;
(e) a BBD29_RS07535 variant polypeptide consisting of an amino acid sequence of SEQ ID NO: 19, wherein alanine, an amino acid corresponding to position 203 of SEQ ID NO: 21, is substituted with threonine; and
(f) a BBD29_RS02010 variant polypeptide consisting of an amino acid sequence of SEQ ID NO: 97, wherein arginine, an amino acid corresponding to position 349 of SEQ ID NO: 99, is substituted with glutamine.

6. The strain of claim 4, wherein the strain has an increased L-arginine-producing ability, as compared to *Corynebacterium glutamicum* comprising a polypeptide of SEQ ID NO: 3 or a polynucleotide encoding the same.

7. A method of producing L-arginine, the method comprising the step of culturing, in a medium, a *Corynebacterium glutamicum* strain comprising any one or more of the variant polypeptide of claim 1 and a polynucleotide encoding the variant polypeptide.

8. A composition for producing L-arginine, the composition comprising any one or more of the variant polypeptide of claim 1; and a microorganism comprising the variant polypeptide, a polynucleotide encoding the variant polypeptide, or a combination thereof.

9. Use of a microorganism comprising the variant polypeptide of claim 1, a polynucleotide encoding the variant polypeptide, or a combination thereof in producing L-arginine.
